(19) Europäisches Patentamt
European Patent Office
Office européen des brevets

(11) EP 0 919 223 A1

(12) **EUROPEAN PATENT APPLICATION**
published in accordance with Art. 158(3) EPC

(43) Date of publication:
02.06.1999 Bulletin 1999/22

(21) Application number: 97950412.3

(22) Date of filing: 25.12.1997

(51) Int. Cl.⁶: **A61K 7/48**, A61K 7/00

(86) International application number:
PCT/JP97/04859

(87) International publication number:
WO 98/29093 (09.07.1998 Gazette 1998/27)

(84) Designated Contracting States:
DE FR GB IT NL

(30) Priority: 27.12.1996 JP 358052/96

(71) Applicant:
SHISEIDO COMPANY, LTD.
Chuo-ku Tokyo (JP)

(72) Inventor:
INOMATA, Shinji,
The Shiseido Research Center
Yokohama-shi, Kanagawa 223 (JP)

(74) Representative:
Rinuy, Santarelli
14, avenue de la Grande Armée
75017 Paris (FR)

(54) **ANTIAGING AGENT**

(57)     This invention relates to an anti-aging agent, more particularly, it relates to an anti-aging agent having an elastase inhibiting action and capable of maintaining the tautness and elasticity of the skin and maintaining a youthful state of the skin.

According to the present invention, there is provided an anti-aging agent containing, as an effective ingredient, a solvent extract of Uncaria gambir Roxburgh.

Fig. 1

## Description

TECHNICAL FIELD

The present invention relates to an anti-aging agent. More specifically, it relates to an anti-aging agent having an elastase inhibiting action and capable of maintaining the tautness and elasticity of the skin and maintaining a youthful state of the skin. The anti-aging agent of the present invention can be suitably used for basic cosmetics and also for makeup cosmetics, hair cosmetics, bath additives, etc.

BACKGROUND ART

In the past, consideration had been given to the need for anti-aging agents. However, since the mechanism, definition, etc. of aging had not been made clear, in general it had been generally judged by measuring the state of moisture retention as the lushness of the skin or measuring the elasticity of the skin or by visually observing the color of the skin.

However, in recent years, various studies on aging have been progressed. As the causes for aging of the skin, viewed macroscopically, actual years has been an important factor. Further, drying, oxidation, the effects by sunlight (UV rays), etc. have been mentioned as direct factors relating to skin aging.

As specific phenomenon of skin aging, the decrease in the collagen and elastin in the dermis, the decrease in the hyaluronic acid and other mucopolysaccharides, damages of cell by UV rays, etc. have been known. Among these, elastin cross-links and contributes to the elasticity of the tissue, but exposure to UV rays and actual aging results in the excessive production of an enzyme destructive elastin, that is, elastase. The degradation of elastin due to this is believed to lead to the decrease in the elasticity of the skin.

Accordingly, it is important for preventing aging of the skin to suppress the activity of elastase and to prevent the degradation of the elastin, which gives elasticity and tautness to the skin.

DISCLOSURE OF INVENTION

Accordingly, the object of the present invention is to provide an anti-aging agent capable of exhibiting the effect of preventing aging of the skin and of maintaining a youthful state of the skin by suppressing the activity of elastase and restoring and maintaining the tautness and elasticity of the skin.

In accordance with the present invention, there is provided an anti-aging agent comprising, as an effective ingredient, a solvent extract of Uncaria gambir Roxburgh.

BRIEF DESCRIPTION OF THE DRAWINGS

Figure 1 is a graph showing the elastase inhibiting activity of an Uncaria gambir Roxburgh extract in comparison with fetal calf serum.

Figure 2 shows the elastase inhibiting activity at low concentration of Uncaria gambir Roxburgh extract.

BEST MODE FOR CARRYING OUT THE INVENTION

The constitution of the present invention will now be explained in detail.

The present inventors investigated the elastase inhibiting activity of a wide variety of substances in view of the above-mentioned problems and, as a result, found that a solvent extract of Uncaria gambir Roxburgh, a plant of the Rubiaceae, has a superior elastase inhibiting activity, whereby the present invention was completed. A solvent extract of Uncaria gambir Roxburgh has long been known to have a stegnotic action and has been used for astrigent stegnosis and, as a medicine for intestinal disorders, for diarrhea, bloody stool, bloody urine, bloody diarrhea etc. In recent years, further, there have been reports of an antioxidation action and a hyaluronidase inhibiting action (JP-A-59-166585 and JP-A-2-11520). However, the application of an Uncaria gambir Roxburgh extract to an anti-aging agent or elastase inhibiting agent has not been known at all.

The Uncaria gambir Roxburgh usable in the present invention is a twining indeciduous shrub naturally occurring and being cultivated in India and Southeast Asia. The extract usable in the present invention is obtained from the dried water soluble extract of the leaves and young branches of the plant immersed or heated and refluxed together with an extraction solvent, filtered, then concentrated.

The extraction solvent which may be used for extracting the Uncaria gambir Roxburgh in the present invention

2

may be any solvent which is ordinarily used for extraction. In particular, alcohols such as methanol, ethanol or 1,3-butylene glycol, hydrous alcohols, acetone, ethyl acetate esters, or other organic solvents may be used alone or in any combination thereof.

The formulating amount of the extract of Uncaria gambir Roxburgh in the present invention is, for example, in an external skin treatment containing an anti-aging agent, 0.0001 to 20.0 % by weight as dried product in the total weight of the external skin treatment, preferably 0.001 to 10.0 % by weight. If the content is less than 0,0001 % by weight, the desired effect of the present invention is not sufficiently exhibited. Contrary to this, if the content is more than 20.0 % by weight, formation into a preparation becomes difficult. Further, even if the content of the Uncaria gambir Roxburgh extract is more than 10.0 % by weight, the further great improvement in effect cannot be obtained.

The Uncaria gambir Roxburgh extract usable in the present invention exhibits a superior elastase inhibiting activity to the human skin, and therefore, an anti-aging agent containing the Uncaria gambir Roxburgh extract can prevent the aging of skin and maintain a youthful, healthy state of the skin.

The anti-aging agent of the present invention may suitably contain therein, if necessary, in addition to the above-mentioned essential ingredient, ingredients which normally can be used for cosmetics and external skin treatments of pharmaceuticals etc., for example, whitening agent, moisture retainers, anti-oxidants, oil ingredients, UV absorbers, surfactants, thickeners, alcohols, powder components, coloring agents, aqueous ingredients, water, various skin nutrients, etc.

As other ingredients, metal sequestering agents such as disodium edetate, trisodium edetate, sodium citrate, sodium polyphosphate, sodium metaphosphate, gluconic acid, medicinal agents such as caffeine, tannin, verapamyl, tranexamic acid and the derivatives thereof, glycyrrhiza extract, grabridin, the hot water extract of the fruit of "Kakyoku", various herbal medicines, tocopherol acetate, glycyrrhizic acid and the derivatives and salts thereof, whitening agent such as vitamin C, magnesium ascorbic phosphate, glucoside ascorbate, arbutin, kojic acid, sugars such as glucose, fructose, mannose, sucrose, trehalose, etc. may also be suitably formulated therein.

The present invention can be applied to a wide range of cosmetics, quasidrugs, etc. used for the external skin application, particularly cosmetics. It may take a broad range of forms such as aqueous solutions, solubilized systems, emulsions, powders, oils, gels, ointments, aerosols, water-oil two-phase systems, water-oil-powder three-phase systems, etc. That is, in the case of basic cosmetics, it may be applied in the above various forms of facial cleansers, lotions, emulsions, creams, gels, essences (beauty lotions), packs, masks. Further, in the case of makeup cosmetics, it may be used for a wide range of types of cosmetics such as foundations while if a toiletry product it may be used for body soap, facial soap, etc. Further, in the case of quasidrugs, it can be used for a wide range of applications such as various ointments. Further, the types or forms of the anti-aging agent of the present invention are not limited to these forms and types.

EXAMPLES

The anti-aging agent of the present invention will now be explained in further detail based on Examples. Nevertheless, the present invention is, of course, not limited to only these Examples. Note that the formulating amounts are indicated in % by weight unless otherwise indicated.

First, the test method of the elastase inhibiting activity of the plant extract according to the present invention and the results thereof are first explained.

Test Example 1

Preparation of Sample

600 g of purified water and 600 g of ethanol were added to 200 g of Uncaria gambir Roxburgh. The mixture was heated at 50°C to dissolve the Uncaria gambir Roxburgh, then was cooled, filtered, and extracted with a solvent to obtain 20 g of an extract. The extract was dissolved in DMSO (dimethyl sulfoxide) at a concentration of 2%. This solution was then diluted to adjust the concentration, which was used in the following test.

Test Method for Elastase Inhibiting Activity and Results Thereof

The elastase activity was measured as follows according to a method of Fujie et al. Further, the test was performed using 0.1M HEPES and 0.5M NaCl (pH 7.4) as a reaction buffer. As the elastase substrate, methoxysuccinyl-alanyl-alanyl-prolyl-valine-p-nitroanilide (BACHEMFEINCHEMIKALIEN AG) was dissolved in DMSO to 80 mM. This was divided into 20 μl portions which were frozen for storage (-80°C). At the time of use, the substrate was diluted with the reaction buffer to 8 mM.

For the elastase, human leukocyte derived elastase (ELASTIN PRODUCT CO., INC.) was used. This was dissolved in the reaction buffer to 200 μg/ml and divided into 10 μl portions which were then frozen for storage (-80°C). At the time of use, the elastase was diluted with the reaction buffer to 5 μg/ml.

25 μl portions of the 8 mM elastase substrate were placed in a 96-well plate (CORNING 25860), then 50 μl of the inhibitor was added. Next, 25 μl of 5 μg/ml elastase was added on ice. The resultant mixture was incubated at 37°C for 20 minutes. Next, the absorbance was measured at 415 nm. The inhibiting rate was based on the following equation:

Inhibiting rate (%) = 100 - (in the presence of an inhibitor/in the absence of an inhibitor) x 100

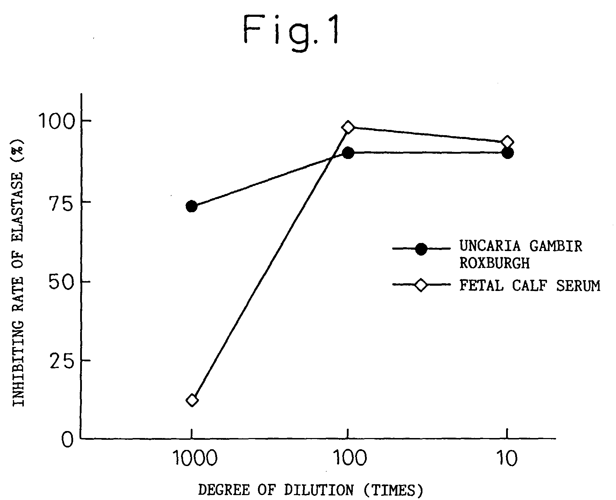

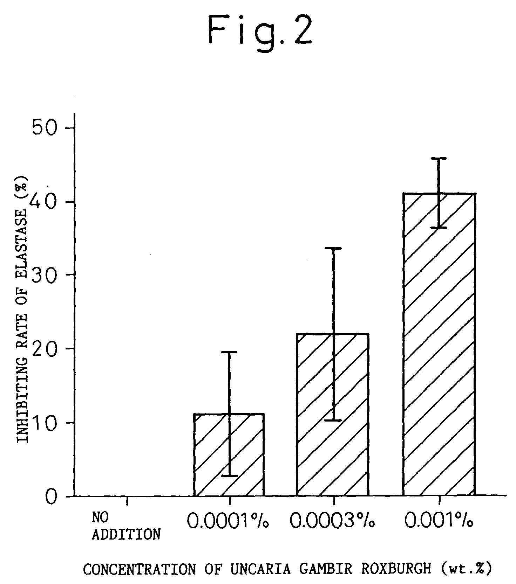

The results are shown in Fig. 1 and Fig. 2. Further, as a Reference Example, a similar test as the above was performed on an in vivo substance exhibiting an elastase inhibiting activity, that is, fetal calf serum (available from GIBCO Co.). The results are also shown in Fig. 1.

Examples of various forms of the anti-aging agent of the present invention will be explained below as Formulation Examples.

Example 1 (Cream)

| (Formulation) | |
|---|---|
| Ingredient | wt% |
| Stearic acid | 3.0 |
| Stearyl alcohol | 5.0 |
| Isopropyl myristate | 18.0 |
| Glyceryl monostearate ester | 3.0 |
| Propylene glycol | 10.0 |
| Uncaria gambir Roxburgh extract | 0.01 |
| Potassium hydroxide | 0.2 |
| Sodium bisulfite | 0.01 |
| Preservative | q.s. |
| Perfume | q.s. |
| Ion exchange water | Balance |

(Preparation Method)

The propylene glycol, Uncaria gambir Roxburgh extract, and potassium hydroxide were added to the ion exchange water and dissolved. The mixture was heated and maintained at 70°C (aqueous phase). The other ingredients were mixed and heated to melt and the resultant mixture maintained at 70°C (oil phase). The oil phase was gradually added to the aqueous phase. The temperature was maintained for a while after all of the ingredients were finished being added so as to allow the reaction to proceed. Next, the resultant mixture was emulsified homogeneously by a homomixer, then was cooled to 30°C while sufficiently stirring.

Example 2 (Cream)

| (Formulation) | |
|---|---|
| Ingredient | wt% |
| Stearic acid | 2.0 |
| Stearyl alcohol | 7.0 |
| Hydrogenated lanolin | 3.0 |
| Squalane | 4.0 |
| 2-Octyldodecyl alcohol | 6.0 |
| Polyoxyethylene(25) cetyl alcohol ether | 3.0 |
| Glyceryl monostearate ester | 2.0 |
| Propylene glycol | 6.0 |
| Uncaria gambir Roxburgh extract | 0.05 |
| Sodium bisulfite | 0.03 |
| Ethylparaben | 0.3 |
| Perfume | q.s. |
| Ion exchange water | Balance |

(Preparation Method)

The propylene glycol was added to the ion exchange water. The mixture was heated and maintained at 70°C (aqueous phase). The other ingredients were mixed and heated to melt and the resultant mixture was maintained at 70°C (oil phase). The oil phase was added to the aqueous phase and preliminarily emulsified. The resultant mixture was homogeneously emulsified by a homomixer, then was cooled to 30°C while sufficiently stirring.

Example 3 (Cream)

| (Formulation) | |
|---|---|
| Ingredient | wt% |
| Solid paraffin | 5.0 |
| Beeswax | 10.0 |
| Vaseline | 15.0 |
| Liquid paraffin | 41.0 |
| Glyceryl monostearate ester | 2.0 |
| Polyoxyethylene(20) sorbitan monolaurate ester | 2.0 |
| Soap powder | 0.1 |
| Borax | 0.2 |
| Uncaria gambir Roxburgh extract | 0.1 |

(continued)

| (Formulation) | |
|---|---|
| Ingredient | wt% |
| Sodium bisulfite | 0.03 |
| Ethylparaben | 0.3 |
| Perfume | q.s. |
| Ion exchange water | Balance |

(Preparation Method)

The soap powder and borax were added to the ion exchange water. The mixture was heated to dissolve them, then held at 70°C (aqueous phase). The other ingredients were mixed and heated to melt and the resultant mixture was maintained at 70°C (oil phase). The oil phase was gradually added to the aqueous phase while stirring to cause a reaction. After the end of the reaction, the resultant mixture was homogeneously emulsified by a homomixer, then was cooled to 30°C while sufficiently stirring.

Example 4 (Emulsion)

| (Formulation) | |
|---|---|
| Ingredient | wt% |
| Stearic acid | 2.5 |
| Cetyl alcohol | 1.5 |
| Vaseline | 5.0 |
| Liquid paraffin | 10.0 |
| Polyoxyethylene(10) monooleate ester | 2.0 |
| Polyethylene glycol 1500 | 3.0 |
| Triethanolamine | 1.0 |
| Carboxyvinyl polymer (Brandname: Carbopol 941, B.F. Goodrich Chemical Co.) | 0.05 |
| Uncaria gambir Roxburgh extract | 3.0 |
| Sodium bisulfite | 0.01 |
| Ethylparaben | 0.3 |
| Perfume | q.s. |
| Ion exchange water | Balance |

(Preparation Method)

The carboxyvinyl polymer was dissolved in a small amount of the ion exchange water (A phase). The polyethylene glycol 1500 and triethanolamine were added to the remaining ion exchange water. The mixture was heated to dissolve them, then maintained at 70°C (aqueous phase). The other ingredients were mixed and heated to melt and the resultant mixture was maintained at 70°C (oil phase). The oil phase was added to the aqueous phase and preliminarily emulsified, then the A phase was added and the mixture was homogeneously emulsified by a homomixer. After emulsification, the resultant mixture was cooled to 30°C while sufficiently stirring.

## Example 5 (Emulsion)

| (Formulation) | |
|---|---|
| Ingredient | wt% |
| Microcrystalline wax | 1.0 |
| Beeswax | 2.0 |
| Lanolin | 20.0 |
| Liquid paraffin | 10.0 |
| Squalane | 5.0 |
| Sorbitan sesquioleate ester | 4.0 |
| Polyoxyethylene(20) sorbitan monooleate ester | 1.0 |
| Propylene glycol | 7.0 |
| Uncaria gambir Roxburgh extract | 10.0 |
| Sodium bisulfite | 0.01 |
| Ethylparaben | 0.3 |
| Perfume | q.s. |
| Ion exchange water | Balance |

(Preparation Method)

The propylene glycol was added to the ion exchange water. The mixture was heated, then maintained at 70°C (aqueous phase). The other ingredients were mixed and heated to melt and the resultant mixture was maintained at 70°C (oil phase). The aqueous phase was gradually added to the oil phase while stirring and the mixture was homogeneously emulsified by a homomixer. After emulsification, the resultant mixture was cooled to 30°C while sufficiently stirring.

## Example 6 (Gel)

| (Formulation) | |
|---|---|
| Ingredient | wt% |
| 95% Ethyl alcohol | 10.0 |
| Dipropylene glycol | 15.0 |
| Polyoxyethylene (50) oleyl alcohol ether | 2.0 |
| Carboxyvinyl polymer (Brandname: Carbopol 940, B.F. Goodrich Chemical Co.) | 1.0 |
| Sodium hydroxide | 0.15 |
| L-Arginine | 0.1 |
| Uncaria gambir Roxburgh extract | 7.0 |
| Sodium 2-hydroxy-4-methoxybenzophenone sulfonate | 0.05 |

(continued)

| (Formulation) | |
|---|---|
| Ingredient | wt% |
| Ethylenediamine tetraacetate・3Na・2H$_2$O | 0.05 |
| Methylparaben | 0.2 |
| Flavoring agent | q.s. |
| Ion exchange water | Balance |

(Preparation Method)

The Carbopol 940 was homogeneously dissolved in the ion exchange water. On the other hand, the Uncaria gambir Roxburgh extract and polyoxyethylene(50)oleyl alcohol ether were dissolved in 95% ethanol and added to the aqueous phase. Next, the other ingredients were added, then the mixture was neutralized and thickened by the sodium hydroxide and L-arginine.

Example 7 (Essence)

| (Formulation) | |
|---|---|
| Ingredient | wt% |
| (A phase) | |
| Ethyl alcohol (95%) | 10.0 |
| Polyoxyethylene(20) octyldodecanol | 1.0 |
| Pantothenyl ethyl ether | 0.1 |
| Uncaria gambir Roxburgh extract | 1.5 |
| Methylparaben | 0.15 |
| (B phase) | |
| Potassium hydroxide | 0.1 |
| (C phase) | |
| Glycerol | 5.0 |
| Dipropylene glycol | 10.0 |
| Sodium bisulfite | 0.03 |
| Carboxyvinyl polymer (brandname: Carbopol 940, B.F. Goodrich Chemical Co.) | 0.2 |
| Purified water | Balance |

(Preparation Method)

The A phase and C phase were respectively homogeneously dissolved, then the A phase was added to the C phase to solubilize it. Next, the B Phase was added, then the container was filled therewith.

Example 8 (Pack)

| (Formulation) | |
|---|---|
| Ingredient | wt% |
| (A phase) | |
| Dipropylene glycol | 5.0 |
| Polyoxyethylene(60) hydrogenated castor oil | 5.0 |
| (B phase) | |
| Uncaria gambir Roxburgh extract | 0.01 |
| Olive oil | 5.0 |
| Tocopherol acetate | 0.2 |
| Ethylparaben | 0.2 |
| Perfume | 0.2 |
| (C phase) | |
| Sodium bisulfite | 0.03 |
| Polyvinyl alcohol (saponification value 90, polymerization degree 2,000) | 13.0 |
| Ethanol | 7.0 |
| Purified water | Balance |

(Preparation Method)

The A phase, B phase, and C phase were respectively homogeneously dissolved, then the B phase was added to the A phase to solubilize it. Next, the C phase was added, then the container was filled therewith.

Example 9 (Solid Foundation)

| (Formulation) | |
|---|---|
| Ingredient | wt% |
| Talc | 43.1 |
| Kaolin | 15.0 |
| Sericite | 10.0 |
| Zinc white | 7.0 |
| Titanium dioxide | 3.8 |
| Yellow iron oxide | 2.9 |
| Black iron oxide | 0.2 |
| Squalane | 8.0 |
| Isostearic acid | 4.0 |

(continued)

| (Formulation) | |
|---|---|
| Ingredient | wt% |
| Monooleate POE sorbitan | 3.0 |
| Isocetyl octanate | 2.0 |
| Uncaria gambir Roxburgh extract | 1.0 |
| Preservative | q.s. |
| Perfume | q.s. |

(Preparation Method)

The powder ingredients, that is, the talc to black iron oxide, were fully mixed by a blender. The oil ingredients of the squalane to the isocetyl octanate, the Uncaria gambir Roxburgh extract, the preservative, and the flavoring agent were added thereto, the resultant mixture was kneaded well, then filled into a container and shaped.

Example 10 (Emulsion Type (Cream Type) Foundation)

| (Formulation) | |
|---|---|
| Ingredient | wt% |
| (Powder portion) | |
| Titanium dioxide | 10.3 |
| Sericite | 5.4 |
| Kaolin | 3.0 |
| Yellow iron oxide | 0.8 |
| Red iron oxide | 0.3 |
| Black iron oxide | 0.2 |
| (Oil phase) | |
| Decamethylcyclopentasiloxane | 11.5 |
| Liquid paraffin | 4.5 |
| Polyoxyethylene modified dimethylpolysiloxane | 4.0 |
| (Aqueous phase) | |
| Purified water | 50.0 |
| 1,3-Butylene glycol | 4.5 |
| Uncaria gambir Roxburgh extract | 1.5 |
| Sorbitan sesquioleate ester | 3.0 |
| Preservative | q.s. |
| Perfume | q.s. |

(Preparation Method)

The aqueous phase was heated and stirred, then the fully mixed and pulverized powder portion was added and the two processed by a homomixer. The heated and mixed oil phase was added and the resultant mixture was processed by a homomixer, then the flavoring agent was added while stirring and the resultant mixture was cooled to room temperature.

Comparative Example 1

The foundation prepared by replacing the Uncaria gambir Roxburgh extract in the formulation of Example 10 with water was used as Comparative Example 1.
The following monitor test was carried out for the cosmetics obtained in Example 10 and Comparative Example 1. The results are shown in Table 1.

Monitor Test

One hundred healthy adult women having an age 25 to 57 extracted at random were used as test subjects. The cosmetics were used on the skin of the face every day for one month, then the effect of improvement on the large wrinkles and small wrinkles was checked.

(a) Effect of Improvement on Large Wrinkles and Small Wrinkles

The state of the skin was visually observed and evaluated, based on the following evaluation criteria.

(Evaluation Criteria)

A:      Completely disappeared
B:      Somewhat reduced
C:      No change
D:      Slightly increased
E:      Increased

(b) Effect on Tautness and Sagging of Skin

The state of the skin was observed visually and evaluated based on the following evaluation criteria.

(Evaluation Criteria)

A:      Extremely improved
B:      Improved
C:      No change
D:      Became somewhat noticable
E:      Became noticable

Note that, in the monitor test, when using the cosmetics obtained in Example 10 and Comparative Example 1, not even one test subject experienced an abnormality of the skin.

Table 1

| | Effect on large wrinkles and small wrinkles (persons) | | | | | Effect on tautness and sagging (persons) | | | | |
|---|---|---|---|---|---|---|---|---|---|---|
| | A | B | C | D | E | A | B | C | D | E |
| Ex. 10 | 20 | 45 | 30 | 5 | 0 | 15 | 33 | 43 | 9 | 0 |
| Comp. Ex. 1 | 0 | 30 | 58 | 8 | 3 | 0 | 9 | 57 | 18 | 16 |

As clear from the results shown in Table 1, in the case of the cosmetic obtained in Example 10, an improvement was observed from the viewpoint of the large wrinkles and small wrinkles and tautness and sagging of the skin, compared with the case of the cosmetic obtained in Comparative Example 1. This shows that formulating of the Uncaria gambir Roxburgh extract as an effective ingredient is extremely effective as a formulation.

INDUSTRIAL APPLICABILITY

As explained above, according to the present invention, by formulating the Uncaria gambir Roxburgh extract as an effective ingredient, it is possible to provide an anti-aging agent exhibiting a superior anti-aging effect. That is, it is possible to provide an anti-aging agent exhibiting the superior cosmetic effect capable of maintaining skin with elasticity and free from wrinkles or sagging and of preventing aging of the skin and maintaining a youthful state of the skin by suppressing conversion of the elastin by an elastase inhibiting activity.

**Claims**

1.  An anti-aging agent comprising, as an effective ingredient, a solvent extract of Uncaria gambir Roxburgh.

2.  An anti-aging agent as claimed in claim 1, which is an elastase inhibitor.

3.  An anti-aging cosmetic composition comprising, as an effective ingredient, a solvent extract of Uncaria gambir Roxburgh.

# Fig.1

# Fig.2

CONCENTRATION OF UNCARIA GAMBIR ROXBURGH (wt.%)

EP 0 919 223 A1

# INTERNATIONAL SEARCH REPORT

International application No.

PCT/JP97/04859

A. CLASSIFICATION OF SUBJECT MATTER
Int.Cl⁶ A61K7/48, 7/00

According to International Patent Classification (IPC) or to both national classification and IPC

B. FIELDS SEARCHED

Minimum documentation searched (classification system followed by classification symbols)
Int.Cl⁶ A61K7/48, 7/00

Documentation searched other than minimum documentation to the extent that such documents are included in the fields searched

Electronic data base consulted during the international search (name of data base and, where practicable, search terms used)
CAPLUS (STN), REGISTRY (STN), WPIDS (STN)

C. DOCUMENTS CONSIDERED TO BE RELEVANT

| Category* | Citation of document, with indication, where appropriate, of the relevant passages | Relevant to claim No. |
|---|---|---|
| PA | JP, 9-87163, A (Shiseido Co., Ltd.),<br>March 31, 1997 (31. 03. 97) (Family: none) | 1-3 |
| PA | JP, 9-87164, A (Shiseido Co., Ltd.),<br>March 31, 1997 (31. 03. 97) (Family: none) | 1-3 |
| PA | JP, 9-87137, A (Shiseido Co., Ltd.),<br>March 31, 1997 (31. 03. 97) (Family: none) | 1-3 |
| X | JP, 2-243632, A (Ichimaru Falcos K.K.),<br>September 27, 1990 (27. 09. 90) (Family: none) | 1, 3 |
| X | JP, 7-17847, A (Sansei Seiyaku K.K.),<br>January 20, 1995 (20. 01. 95) (Family: none) | 1, 3 |
| A | JP, 8-127524, A (Yugen Kaisha Nonokawa Shoji),<br>May 21, 1996 (21. 05. 96) (Family: none) | 1-3 |
| A | JP, 59-216810, A (K.K. Osaka Yakuhin Kenkyusho),<br>December 6, 1984 (06. 12. 84) (Family: none) | 1-3 |

☐ Further documents are listed in the continuation of Box C.   ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search<br>March 3, 1998 (03. 03. 98) | Date of mailing of the international search report<br>March 17, 1998 (17. 03. 98) |
|---|---|
| Name and mailing address of the ISA/<br>Japanese Patent Office | Authorized officer |
| Facsimile No. | Telephone No. |

Form PCT/ISA/210 (second sheet) (July 1992)

15